# EUROPEAN PATENT SPECIFICATION

(11) **EP 1 528 052 B1**
(45) Date of publication and mention of the grant of the patent: **31.07.2013**
(21) Application number: 04025390.8
(22) Date of filing: 26.10.2004
(51) Int. Cl.: C07C 17/12, C07C 25/02, C07C 17/20, C07C 22/08, C07C 209/10, C07C 211/52

(54) **Process for the production of 4-amino-3,5-dichlorobenzotrifluoride with high purity commercial products being obtained in parallel**
Verfahren zur Parallelherstellung von 4-amino-3,5-dichloro-benzotrifluorid und hochreinen kommerziellen Produkten
Procédé pour la préparation de 4-amino-3,5-dichloro-benzotrifluoride et des produits commerciaux de haut degré de pureté en parallèle

(30) Priority: 29.10.2003 IT TO20030850
(43) Date of publication of application: 04.05.2005
(73) Proprietor: FINCHIMICA S.p.A., 25025 Manerbio (Brescia) (IT)
(72) Inventor: Pastorio, Andrea, 26034 Piadena (Cremona) (IT); Mollica, Daniela, 25062 Concesio (Brescia) (IT); Ravetta, Giacomo, 25050 Monte Isola (Brescia) (IT); Mancini, Gianluca, 25024 Leno (Brescia) (IT)
(74) Representative: Rambelli, Paolo

(56) References cited:
- GB-A- 1 535 149
- US-A- 3 950 445
- US-A- 4 242 286
- US-A- 4 769 503
- US-B1- 6 479 703
- DATABASE BEILSTEIN [Online] BEILSTEIN INSTITUTE FOR ORGANIC CHEMISTRY, FRANKFURT-MAIN, DE; XP002309824 retrieved from XFIRE accession no. RID2973011 & USHAKOV, A. ; ALIKHANOV, P.; MOTSAREV, G. ET AL: J. ORG. CHEM. USSR, vol. 20, 1984,
- DATABASE BEILSTEIN [Online] BEILSTEIN INSTITUTE FOR ORGANIC CHEMISTRY, FRANKFURT-MAIN, DE; XP002309825 retrieved from XFIRE accession no. RID2973010 & USHAKOV, A.; ALIKHANOV, P.; MOTSAREV, G. ET AL.: J. ORG. CHEM. USSR, vol. 20, 1984,
- DATABASE BEILSTEIN [Online] BEILSTEIN INSTITUTE FOR ORGANIC CHEMISTRY, FRANKFURT-MAIN, DE; XP002309826 retrieved from XFIRE accession no. RID2973009 & USHAKOV, A.; ALIKHANOV, P.; MOTSAREV, G. ET AL.: J. ORG. CHEM. USSR, vol. 20, 1984, pages 1993-1996,
- DATABASE BEILSTEIN [Online] BEILSTEIN INSTITUTE FOR ORGANIC CHEMISTRY, FRANKFURT-MAIN, DE; XP002309827 retrieved from XFIRE accession no. RID358797 & BOOTH; ELSEY; BURCHFIELD: J. AMER. CHEM. SOC., vol. 57, 1935, pages 2064-2065,
- DATABASE BEILSTEIN [Online] BEILSTEIN INSTITUTE FOR ORGANIC CHEMISTRY, FRANKFURT-MAIN, DE; XP002309828 retrieved from XFIRE accession no. RID278129 & MARYOTT; HOBBS; GROSS: J. AMER. CHEM. SOC., vol. 62, 1940, page 2320,

## Description

The present invention relates to a process for the production of 4-amino-3,5-dichlorobenzotrifluoride (2,6-dichloro-4-trifluoromethylaniline) by an amination reaction of 3,4,5-trichlorobenzotrifluoride.

4-Amino-3,5-dichlorobenzotrifluoride is an important intermediate for the synthesis of phytopharmaceuticals.

US 4 096 185 describes the amination reaction of halogenated aromatic compounds, in particular 4-chlorobenzotrifluoride, for the production of the corresponding 4-amino derivative. In said process, the amination reaction is performed with ammonia in a non-aqueous solvent and in the presence of a catalyst system comprising a copper compound (in particular a cupric or cuprous halide) and an alkali metal halide or alkaline earth metal halide under a pressure of 3.04 to 40.5 MPa (30 to 400 atmospheres) and at a temperature of between 150°C and 240°C.

US 6 479 703 describes a similar amination reaction with ammonia of polyhalogenated benzotrifluoride derivatives for the production of 3,5-dihalo-para-trifluoromethylanilines, including 4-amino-3,5-dichlorobenzotrifluoride, starting from 3,4,5-trichlorobenzotrifluoride; the reaction is performed with ammonia at a temperature of between 180°C and 350°C, optionally in the presence of a catalyst consisting of an alkali metal halide.

The production of 3,4,5-trichlorobenzotrifluoride, which is used as the starting material in US 6 479 703, is for example described in EP-A-0 150 587, where the 3,4,5-trichlorobenzotrifluoride is obtained by means of chlorine substitution of m-nitro groups in a nitrobenzotrifluoride compound using gaseous chlorine in the presence of a catalyst comprising a metal salt and a sulfur compound.

Database Beilstein (online), Beilstein Institute for Organic Chemistry, Frankfurt-Main DE; XFIRE accession No. RID 278129 describes the preparation of 4-chlorobenzotrichloride by photochlorination of parachlorotoluene with gaseous chlorine.

The present invention provides a novel process for the production of 4-amino-3,5-dichlorobenzotrifluoride via the amination of 3,4,5-trichlorobenzotrifluoride (3,4,5-TCBTF) which proves economically advantageous and convenient for industrial implementation.

This process does not require costly purification stages and nevertheless permits the obtainment of a high purity product, unlike conventional commercial 2,6-dichloro-4-trifluoromethylaniline currently produced by other means. Another peculiarity of this invention is that the product yields high grade 3,4-DCBTF as a commercially valuable secondary product.

The process which is the subject matter of the invention is stated in the claims which follow.

In the attached drawings:
- Fig. 1 is a flow diagram which illustrates, by way of non-limiting example, a sequence of the stages in the process according to the invention;
- Fig. 2 is a diagram showing the chlorination kinetics of 4-chlorobenzotrichloride.

In the process according to the invention, 3,4,5-trichlorobenzotrifluoride, which is intended to be subjected to amination, is prepared by means of the following process stages.
A) Ring chlorination of 4-chlorobenzotrichloride (PCBTC) in the presence of a Lewis acid in order to obtain a mixture of 3,4-dichlorobenzotrichloride (3,4-DCBTC) and 3,4,5-trichlorobenzotrichloride (3,4,5-TCBTC), together with possible minor quantities of other chlorinated derivatives, such as 2,4,5-trichlorobenzotrichloride (2,4,5-TCBTC), 2,3,4,5-tetrachlorobenzotrichloride (2,3,4,5-TCBTC) and possibly other chlorinated compounds.
   The PCBTC used as the starting compound in this stage may conveniently be obtained by means of free-radical chlorination of the corresponding parachlorotoluene (PCT). Free-radical chlorination is generally performed by reacting PCT with gaseous chlorine at elevated temperature and/or in the presence of ultraviolet light or another catalyst (c.f., in the attached flow diagram, the photochlorination stage A₀).
   It is, however, understood that PCBTC may be produced by means of any other known prior art method.
   Ring chlorination of PCBTC, which is referred to in stage A, is a per se known reaction, described for example in GB 1 535 149 and in US 4 769 503.
   The process of GB 1 535 149 is intended for the selective production of 3,4-dichloro-, 3,4,5-trichloro-, 2,4,5-trichloro- or 2,3,4,5-tetrachlorobenzotrichloride derivatives, or of a mixture thereof from which the individual reaction products may be separated by distillation or crystallisation.
   The process is performed at a temperature which is preferably between 10°C and 100°C and preferably under standard pressure in the presence of catalytic quantities of ferric chloride or aluminium chloride with gaseous chlorine or by using an agent which releases chlorine.
   The process of US 4 769 503 is intended for the production of 3,4-dichlorobenzotrihalides and proceeds at a temperature of 20°C to 120°C with a Lewis acid catalyst and with the use of gaseous chlorine.
   Ring chlorination of PCBTC proceeds in accordance with consecutive reaction kinetics with initial formation of 3,4-DCBTC, the generation of which reaches a maximum once the PCBTC reactant has been virtually completely consumed.
   As chlorination proceeds, 3,4-DCBTC becomes a primary reactant, giving rise to the formation of the other above-stated chlorinated derivatives.

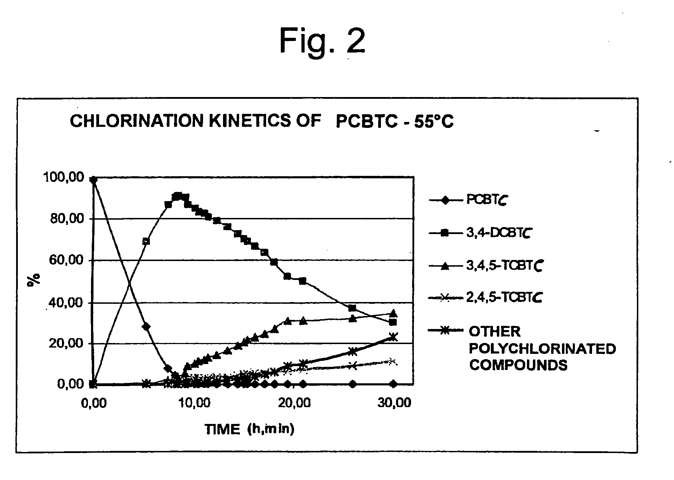

   Fig. 2 is a diagram which schematically illustrates the chlorination kinetics of the PCBTC, determined experimentally, in the context of the testing relating to the process according to the invention under the following conditions:
   - chlorine flow rate: 0.12 mol/mol of PCBTC·h
   - temperature: 55-56°C
   - anhydrous FeCl₃ catalyst: 200 mg/kg.
   Although, as stated above, the chlorination reaction is known per se, stage A) of the process according to the invention is characterised in that the reaction is performed until a mixture of chlorinated reaction products is obtained which has a 3,4,5-TCBTC concentration between greater than 10% and 30% by weight and preferably of between 20% and 30% by weight and corresponds to a concentration of 3,4-TCBTC comprised between less than 90% and 55% by weight, preferably less than 80% and most preferably of between 75% and 55% by weight, relative to the total weight of the chlorinated derivatives in the reaction mixture.
   In molar terms, the reaction is performed until a molar ratio between 3,4-DCBTC and 3,4,5-TCBTC is obtained which is preferably between 5:1 and 1:1.
   Obtaining the above-stated weight or molar ratios between 3,4-DCBTC and 3,4,5-TCBTC may readily be achieved by apportioning the total number of moles of the chlorine agent, introduced into the reaction mixture, preferably at a constant flow rate, and adjusting the total reaction time accordingly.
   The reaction conditions described above in the cited prior art are applicable to the process according to the invention. The catalyst is preferably anhydrous ferric chloride or ferric chloride hexahydrate and is used in catalytic quantities of generally between 0.1 and 50 mmol per mol of PCBTC.
   The reaction is preferably performed in the absence of solvent and at atmospheric pressure for typical times of the order of 8-20 hours, or with apportionment of chlorine of between 1.1 and 5 mol per mol of PCBTC.
   According to another feature of the process, the reaction mixture is not subjected to separation of the reaction products and the catalyst, but is used directly in the following fluorination stage.
B) Fluorination with hydrofluoric acid of the mixture of chlorination products as obtained in stage A) in order to obtain the corresponding benzotrifluorides.
   The fluorination reaction for production of benzotrifluorides from the corresponding benzotrichlorides is known per se and is described, for example, in US 3 950 445 and US 4 242 286.
   In the process according to the invention, the mixture of benzotrichlorides, originating from stage A), is passed directly into the fluorination reactor, in which the fluorination reaction is performed with vigorous stirring using anhydrous hydrofluoric acid at a temperature of between 15°C and 150°C and under a pressure of 0.1 to 30.04 MPa (1 to 30 atm). The reaction does not require catalysts, but may be performed under gentler conditions by using catalytic quantities of Lewis acids, in particular antimony halides.
   The hydrochloric acid arising from the reaction is absorbed in water and recovered for commercial use.
C) Distillation of the mixture of benzotrifluorides obtained in stage B).
   According to a first variant C₁) of the process according to the invention, distillation of the mixture of benzotrifluorides from stage B) is intended to separate 3,4,5-trichlorobenzotrifluoride (3,4,5-TCBTF) which is to be subjected to amination in the subsequent stage D).
   Separation by distillation, performed at this stage in the overall process, is particularly advantageous in that the benzotrifluorides exhibit substantially lower boiling points than the corresponding benzotrichlorides and moreover have more pronounced differences in stability.
   Taking account of the relative weight ratios between 3,4-DCBTC and 3,4,5-TCBTC in stage A), the distillation stage also brings about the separation of a preponderant quantity of 3,4-DCBTF, which is a valuable product intended for sale. In particular, the process for obtaining 3,4-DCBTF makes it possible to obtain a commercial product of very high purity, indeed the product contains virtually none of the 2,4-DCBTF isomer, which is inevitably formed when 3,4-DCBTF is obtained by ring chlorination of PCBTF; since the market requires 3,4-DCBTF with a low content of 2,4-DCBTF, the conventional method requires a distillation step which is costly in terms of both energy and process yield. Small quantities of 2,4,5-TCBTF, which is intended for disposal, and of 2,3,4,5-tetraCBTF, which is intended for disposal or sale, and small quantities of fluorinated tars are also obtained.

Alternatively, according to variant C₂), the distillation stage is performed to separate 3,4-DCBTF and an isomeric mixture of 3,4,5-TCBTF and 2,4,5-TCBTF which is subjected to amination in the subsequent stage D).

In particular, the process according to the invention is advantageous in that, by means of appropriate selection of the degree of conversion of PCBTC into 3,4-DCBTC and 3,4,5-TCBTC in stage A), it makes it possible to control the quantity of commercially valuable products, in particular 3,4-DCBTF and 3,4,5-TCBTF, the latter being intended for amination, in accordance with commercial requirements.

In this manner, a process is provided which is particularly flexible and economically advantageous and gives rise to a minimal quantity of products which will simply be disposed of.

### D) Amination of 3,4,5-TCBTF.

As already stated, the amination stage is performed by means of per se known processes, described in US 4 096 185 and US 6 479 703, by using ammonia, typically at temperatures of between 150°C and 300°C, in a polar organic solvent, such as in particular N-methylpyrrolidone, or by using the 3,4,5-TCBTF itself as solvent, optionally in the presence of a catalyst consisting of an alkali metal halide.

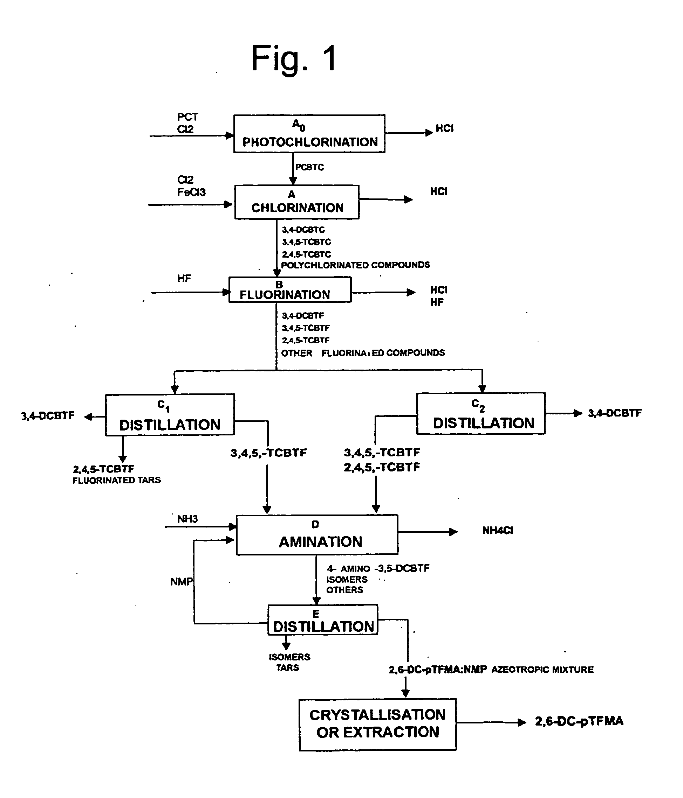

On completion of the amination reaction, the reaction mixture is filtered off from the ammonium chloride produced and the liquid phase is vacuum distilled (stage E) - Fig. 1), in order to obtain 4-amino-3,5-DCBTF, with separation of the higher boiling isomer thereof which has the amino group in meta position.

As already stated, the process may be made still more advantageous in terms of energy yields and purification by avoiding the difficult separation of the isomers 3,4,5-TCBTF and 2,4,5-TCBTF in stage C) and passing the mixture of the two for amination, as shown in the flow diagram of Fig. 1 with stage C₂).

Vacuum distillation of 4-amino-3,5-DCBTF in order to separate it from the meta isomer thereof also results in the separation of the aminated product derived from 2,4,5-TCBTF because the latter is higher boiling.

If N-methylpyrrolidone is used as the solvent during amination, distillation gives rise to an azeotropic mixture, from which the 4-amino-3,5-DCBTF (or 2,6-DC-pTFMA) may be obtained by crystallisation or extraction.

The following examples of embodiment describe the process according to the invention in greater detail.

### Example 1a - Chlorination

5 mol (1150 g) of 4-chlorobenzotrichloride (99% pure) and 2 mmol (0.323 g) of iron trichloride are placed in a 1 1, jacketed glass reactor equipped with a stirrer. Under a stream of nitrogen, the reactor is adjusted to a temperature of 55°C and, with stirring, the stream of nitrogen is replaced with a stream of 0.55 mol/h (39 g/h) of gaseous chlorine.

After 800 minutes, 7.33 mol (520.4 g) of chlorine have been supplied and the stream is shut off. After having been degassed with nitrogen, the reaction mixture is cooled, discharged and analysed by gas chromatography. The mixture (1381 g in total) has the following composition by weight:
- 3,4-dichlorobenzotrichloride 68.9%
- 3,4,5-trichlorobenzotrichloride 21.35%
- 2,4,5-trichlorobenzotrichloride 4.7%
- 2,3,4,5-tetrachlorobenzotrichloride 3.10%.

### Examples 1b-1c - Fluorination and separation by distillation

The mixture of benzotrichlorides from Example 1a is passed directly to the fluorination reactor, which consists of an AISI 316 steel autoclave equipped with a stirrer and connected via a valve for pressure control to the gaseous hydrogen chloride absorption system.

Once the mixture of benzotrichlorides has been charged into the autoclave, it is cooled to 5°C and 25.5 mmol (510 g) of anhydrous hydrofluoric acid are added. While the mixture is subjected to vigorous stirring in such a manner as to permit the two phases to react, it is heated until it reaches a final temperature of 120°C which is maintained for 2 hours.

The pressure within the autoclave is maintained at 20 bar by means of a control valve and the stream of gaseous HCl leaving the autoclave (15 mol, 547.5 g) is absorbed in water to produce 32% HCl for commercial use.

When the production of gaseous HCl has ceased and the pressure remains constant, the reaction is complete. The temperature is lowered, stirring is stopped and the phases are separated in appropriate apparatus.

The resultant organic phase (5 mol, 1133.5 g) is vacuum distilled to obtain 3.58 mol (768 g) of 3,4-dichlorobenzotrifluoride of a purity of 99.5% (yield 71.5%) and 1.07 mol (268 g) of 3,4,5-trichlorobenzotrifluoride of a purity of 99% (yield 21.4%). The remaining 98 g consist of 2,4,5-trichlorobenzotrifluoride and 2,3,4,5-tetrachlorobenzotrifluoride and remain as heavy bottoms products.

### Examples 1b-1c (version 2) - Fluorination and separation by distillation

The description given for the first version remains valid with the following differences.

The resultant organic phase (5 mol, 1133.5 g) is vacuum distilled to obtain 3.58 mol (768 g) of 3,4-dichlorobenzotrifluoride of a purity of 99.5% (yield 71.5%) and 1.34 mol (334 g) of a mixture consisting of 1.07 mol (268 g) of 3,4,5-trichlorobenzotrifluoride and 0.27 mol (66 g) of 2,4,5-trichlorobenzotrifluoride. A further 31.5 g of polychlorobenzotrifluorides remain as heavy bottoms products.

### Example 1d - Amination

0.5 mol (124.7 g) of 3,4,5-trichlorobenzotrifluoride and 1.8 mol (178 g) of N-methylpyrrolidone are mixed in an autoclave. The autoclave is cooled to 0°C and charged with 4 mol of ammonia (68 g) and heated with vigorous stirring to 190°C for 8 hours.

After cooling and removal of excess ammonia, the reaction mixture is filtered off from the ammonium chloride produced and the liquid phase is vacuum distilled. The distillation yields 110.4 g of an azeotropic mixture containing 83% 2,6-dichloro-4-trifluoromethylaniline and 17% N-methylpyrrolidone.

The 99% pure product is precipitated out from the azeotropic mixture by addition of water and, after filtration and drying, 0.396 mol (91 g) of 2,6-dichloro-4-trifluoromethylaniline (yield 79.2%) are obtained.

### Example 1d (version 2) - Amination

0.5 mol (124.7 g) of an 80:20 isomeric mixture consisting of 3,4,5-trichlorobenzotrifluoride and 2,4,5-trichlorobenzotrifluoride and 1.8 mol (178 g) of N-methylpyrrolidone are introduced into an autoclave. The autoclave is cooled to 0°C and charged with 4 mol of ammonia (68 g) and heated with vigorous stirring to 190°C for 8 hours.

After cooling and removal of excess ammonia, the reaction mixture is filtered off from the ammonium chloride produced and the liquid phase is vacuum distilled. The distillation yields 88.3 g of an azeotropic mixture containing 83% 2,6-dichloro-4-trifluoromethylaniline (2,6-DC-pTFMA) and 17% N-methylpyrrolidone.

The 99% pure product is precipitated out from the azeotropic mixture by addition of water and, after filtration and drying, 0.317 mol (73 g) of 2,6-dichloro-4-trifluoromethylaniline (yield 79.2% relative to 3,4,5-TCBTF) are obtained.

Examples 2 and 3 below are given in order to illustrate the advantage of keeping the conversion of 3,4-DCBTC into 3,4,5-TCBTC at a low level in the chlorination stage, in that if conversion of the 3,4-DCBTC is boosted, the result is not a greater yield of 3,4,5-TCBTC, but instead the formation of isomeric and polychlorinated products which require costly disposal.

### Example 2

In the reaction described in Example 1, the stream of chlorine is maintained for 1300 minutes, so apportioning a total of 11.9 mol (846 g) of chlorine. The composition by weight of the resultant mixture is as follows:
- 3,4-dichlorobenzotrichloride 29.8%
- 3,4,5-trichlorobenzotrichloride 34.8%
- 2,4,5-trichlorobenzotrichloride 11%
- 2,3,4,5-tetrachlorobenzotrichloride 22.8%.

### Example 3

In the reaction described in example 1, the stream of chlorine is maintained for 1600 minutes, so apportioning a total of 14.67 mol (1042 g) of chlorine. The composition by weight of the resultant mixture is as follows:
- 3,4-dichlorobenzotrichloride 9.8%
- 3,4,5-trichlorobenzotrichloride 26.7%
- 2,4,5-trichlorobenzotrichloride 11.9%
- 2,3,4,5-tetrachlorobenzotrichloride 50.3%.

## Claims

1. A process for the production 4-amino-3,5-dichlorobenzotrifluoride by amination of 3,4,5-trichlorobenzotrifluoride, **characterised in that** it comprises the stages:
A) chlorination of 4-chlorobenzotrichloride, in the presence of a Lewis acid, in order to obtain a mixture of 3,4-dichlorobenzotrichloride and 3,4,5-trichlorobenzotrichloride and other chlorine derivatives by performing the reaction in such a manner as to obtain in the mixture of reaction products a concentration of 3,4,5-trichlorobenzotrichloride comprised between greater than 10% and 30% by weight and a concentration of 3,4-dichlorobenzotrichloride comprised between less than 90% and 55% by weight, relative to the total weight of the chlorinated derivatives;
B) fluorination with hydrofluoric acid of the mixture of chlorination products as obtained in stage A) in order to obtain the corresponding benzotrifluorides; and
C) distillation of the mixture of products from stage B) in order to obtain 3,4,5-trichlorobenzotrifluoride or an isomeric mixture of 3,4,5- and 2,4,5-triclorobenzotrifluoride and to separate 3,4-dichlorobenzotrifluoride which is recovered; and
D) amination of 3,4,5-trichlorobenzotrifluoride or of said isomeric mixture.

2. A process according to claim 1, **characterised in that** said chlorination stage A) is performed with the use of gaseous chlorine, or a chlorine-releasing agent, in the presence of anhydrous ferric chloride or ferric chloride hexahydrate.

3. A process according to claim 2, **characterised in that**, in said stage A), ferric trichloride is used in quantities of between 0.1 and 50 mmol per mol of 4-chlorobenzotrichloride.

4. A process according to claims 2 or 3, **characterised in that** said chlorination stage A) is performed at a temperature of between 0°C and 180°C.

5. A process according to any one of claims 1 to 4, **characterised in that** said chlorination stage is performed by means of apportioning a total quantity of chlorine of between 1.1 and 5.0 mol per mol of 4-chlorobenzotrichloride.

6. A process according to any one of claims 1 to 5, **characterised in that** said fluorination stage B) is performed on the chlorination mixture A) as it stands with the use of hydrofluoric acid at a temperature of between 15°C and 180°C, with the use of Lewis acid catalysts.

7. A process according to any one of the preceding claims, **characterised in that** said distillation separation stage C) is performed in order to separate 3,4,5-TCBTF, which is intended to be subjected to amination, and 3,4-DCBTF, which is intended for commercial use.

8. A process according to any one of claims 1 to 6, **characterised in that** said distillation stage C) is performed in order to separate an isomeric mixture of 3,4,5-TCBTF and 2,4,5-TCBTF and in which said isomeric mixture is subjected to amination D).

9. A process according to claim 8, **characterised in that** the amination product of stage D) is subjected to distillation in order to separate 4-amino-3,5-dichlorobenzotrifluoride.

10. A process according to any one of the preceding claims, **characterised in that** the amination reaction D) is performed with ammonia at a temperature of between 150°C and 350°C, optionally in the presence of an alkali metal halide catalyst.

11. A process according to claim 10, **characterised in that** the amination reaction is performed in a polar organic solvent or using the 3,4,5-TCBTF itself as the solvent.

12. A process according to any one of the preceding claims, **characterised in that** the 4-chlorobenzotrichloride, used as a reactant in stage A), is obtained by photochlorination of parachlorotoluene with gaseous chlorine.

13. A process according to any one of the preceding claims **characterised in that** stage A) is carried out so as to obtain in the mixture of reaction products a quantity of 3,4,5-trichlobenzotrichloride of between 20% and 30% by weight and a concentration of 3,4-dichlorobenzotrichloride of between 55 and 75% by weight relative to the total weight of the chlorinated derivatives.

14. A process according to claim 6 **characterised in that** said fluorination stage B) is carried out with the use of antimony halides as the catalyst.

## Patentansprüche

1. Verfahren zur Herstellung von 4-Amino-3,5-dichlorbenzotrifluorid durch Aminierung von 3,4,5-Trichlorbenzotrifluorid, **dadurch gekennzeichnet, dass** es die Stufen umfasst:
A) Chlorierung von 4-Chlorbenzotrichlorid in der Gegenwart einer Lewis-Säure, um eine Mischung von 3,4-Dichlorbenzotrichlorid und 3,4,5-Trichlorbenzotrichlorid und anderen Chlor-Derivaten zu erhalten, mittels Durchführen der Reaktion in solch einer Art, um in der Mischung der Reaktionsprodukte eine Konzentration von 3,4,5-Trichlorbenzotrichlorid, umfasst zwischen größer als 10 Gewichtsprozent und 30 Gewichtsprozent, und eine Konzentration von 3,4-Dichlorbenzotrichlorid, umfasst zwischen weniger als 90 Gewichtsprozent und 55 Gewichtsprozent, zu erhalten, bezogen auf des Gesamtgewicht der chlorierten Derivate;
B) Fluorierung der wie in Stufe A) erhaltenen Mischung der Chlorierungsprodukte mit Fluorwasserstoffsäure, um die entsprechenden Benzotrifluoride zu erhalten; und
C) Destillation der Mischung der Produkte aus Stufe B), um 3,4,5-Trichlorbenzotrifluorid oder eine isomere Mischung von 3,4,5- und 2,4,5-Trichlorbenzotrifluorid zu erhalten, und 3,4-Dichlorbenzotrifluorid zu trennen, welches zurückgewonnen wird; und
D) Aminierung von 3,4,5-Trichlorbenzotrifluorid oder von der isomeren Mischung.

2. Verfahren nach Anspruch 1, **dadurch gekennzeichnet, dass** die Chlorierungs-Stufe A) mittels Verwendung von gasförmigem Chlor oder einem Chlor-freisetzenden Mittel in der Gegenwart von wasserfreiem Eisenchlorid oder Eisenchlorid-Hexahydrat durchgeführt wird.

3. Verfahren nach Anspruch 2, **dadurch gekennzeichnet, dass** in der Stufe A) Eisentrichlorid in Mengen zwischen 0,1 und 50 mmol pro mol von 4-Chlorbenzotrichlorid verwendet wird.

4. Verfahren nach Anspruch 2 oder 3, **dadurch gekennzeichnet, dass** die Chlorierungs-Stufe A) bei einer Temperatur von zwischen 0°C und 180°C durchgeführt wird.

5. Verfahren nach einem der Ansprüche 1 bis 4, **dadurch gekennzeichnet, dass** die Chlorierungs-Stufe mittels Zuteilung einer Gesamtmenge von Chlor von zwischen 1,1 und 5,0 mol pro mol von 4-Chlorbenzotrichlorid durchgeführt wird.

6. Verfahren nach einem der Ansprüche 1 bis 5, **dadurch gekennzeichnet, dass** die Fluorierungs-Stufe B) mit der unveränderten Chlorierungsmischung A) unter Verwendung von Fluorwasserstoffsäure bei einer Temperatur von zwischen 15°C und 180°C, unter Verwendung von Lewis-Säure-Katalysatoren durchgeführt wird.

7. Verfahren nach einem der vorangehenden Ansprüche, **dadurch gekennzeichnet, dass** die Destillations-Trennungs-Stufe C) durchgeführt wird, um 3,4,5-TCBTF, welches vorgesehen ist, Aminierung unterworfen zu werden, und 3,4-DCBTF, welches für kommerzielle Verwendung vorgesehen ist, zu trennen.

8. Verfahren nach einem der Ansprüche 1 bis 6, **dadurch gekennzeichnet, dass** die Destillations-Stufe C) durchgeführt wird, um eine isomere Mischung von 3,4,5-TCBTF und 2,4,5-TCBTF zu trennen, und in welchem die isomere Mischung Aminierung D) unterworfen wird.

9. Verfahren nach Anspruch 8, **dadurch gekennzeichnet, dass** das Aminierungsprodukt von Stufe D) Destillation unterworfen wird, um 4-Amino-3,5-dichlorbenzotrifluorid zu trennen.

10. Verfahren nach einem der vorangehenden Ansprüche, **dadurch gekennzeichnet, dass** die Aminierungsreaktion D) mit Ammoniak bei einer Temperatur von zwischen 150°C und 350°C durchgeführt wird, gegebenenfalls in der Gegenwart eines AlkalimetallHalogenid-Katalysators.

11. Verfahren nach Anspruch 10, **dadurch gekennzeichnet, dass** die Aminierungsreaktion in einem polaren organischen Lösungsmittel oder unter Verwendung von 3,4,5-TCBTF selbst als dem Lösungsmittel durchgeführt wird.

12. Verfahren nach einem der vorangehenden Ansprüche, **dadurch gekennzeichnet, dass** das 4-Chlorbenzotrichlorid, welches in Stufe A) als Reaktant verwendet wird, durch Fotochlorierung von Parachlortoluol mit gasförmigem Chlor erhalten wird.

13. Verfahren nach einem der vorangehenden Ansprüche, **dadurch gekennzeichnet, dass** Stufe A) dergestalt ausgeführt wird, um in der Mischung der Reaktionsprodukte eine Menge an 3,4,5-Trichlorbenzotrichlorid von zwischen 20 Gewichtsprozent und 30 Gewichtsprozent und eine Konzentration von 3,4-Dichlorbenzotrichlorid von zwischen 55 Gewichtsprozent und 75 Gewichsprozent zu erhalten, bezogen auf das Gesamtgewicht der chlorierten Derivate.

14. Verfahren nach Anspruch 6, **dadurch gekennzeichnet, dass** die Fluorierungs-Stufe B) mittels Verwendung von Antimonhalogeniden als der Katalysator durchgeführt wird.

## Revendications

1. Procédé pour la production de 4-amino-3,5-dichlorobenzotrifluorure par amination de 3,4,5-trichlorobenzotrifluorure, **caractérisé en ce qu'**il comprend les étapes :
A) de chloration de 4-chlorobenzotrichlorure, en présence d'un acide de Lewis, afin d'obtenir un mélange de 3,4-dichlorobenzotrichlorure et de 3,4,5-trichlorobenzotrichlorure et d'autres dérivés du chlore par réalisation de la réaction de sorte d'obtenir dans le mélange des produits réactionnels une concentration de 3,4,5-trichlorobenzotrichlorure comprise entre plus de 10 % et 30 % en masse et une concentration de 3,4-dichlorobenzotrichlorure comprise entre moins de 90 % et 55 % en masse, par rapport à la masse totale des dérivés chlorées ;
B) de fluoration avec de l'acide fluorhydrique du mélange de produits de chloration comme obtenu dans l'étape A) afin d'obtenir les benzotrifluorures correspondants ; et
C) de distillation du mélange des produits de l'étape B) afin d'obtenir du 3,4,5-trichlorobenzotrifluorure ou un mélange isomère de 3,4,5- et de 2,4,5-trichlorobenzotrifluorure et de séparer le 3,4-dichlorobenzotrifluorure qui est récupéré ; et
D) d'amination du 3,4,5-trichlorobenzotrifluorure ou dudit mélange isomère.

2. Procédé selon la revendication 1, **caractérisé en ce que** ladite étape de chloration A) est réalisée avec l'utilisation de chlore gazeux, ou d'un agent libérant du chlore, en présence de chlorure ferrique anhydre ou d'hexahydrate de chlorure ferrique.

3. Procédé selon la revendication 2, **caractérisé en ce que**, dans ladite étape A), le trichlorure ferrique est utilisé dans des quantités entre 0,1 et 50 mmol par mole de 4-chlorobenzotrichlorure.

4. Procédé selon les revendications 2 ou 3, **caractérisé en ce que** ladite étape de chloration A) est réalisée à une température entre 0°C et 180°C.

5. Procédé selon l'une quelconque des revendications 1 à 4, **caractérisé en ce que** ladite étape de chloration est réalisée au moyen d'une distribution d'une quantité totale de chlore entre 1,1 et 5,0 mol par mole de 4-chlorobenzotrichlorure.

6. Procédé selon l'une quelconque des revendications 1 à 5, **caractérisé en ce que** ladite étape de fluoration B) est réalisée sur le mélange de chloration A) lorsqu'il repose avec l'utilisation d'acide fluorhydrique à une température de 15°C à 180°C, en utilisant des catalyseurs d'acides de Lewis.

7. Procédé selon l'une quelconque des revendications précédentes, **caractérisé en ce que** ladite étape de séparation par distillation C) est réalisée afin de séparer 3,4,5-TCBTF, lequel doit être soumis à une amination, et 3,4-DCBTF, lequel est destiné à une utilisation commerciale.

8. Procédé selon l'une quelconque des revendications 1 à 6, **caractérisé en ce que** ladite étape de distillation C) est réalisée afin de séparer un mélange isomère de 3,4,5-TCBTF et de 2,4,5-TCBTF et dans lequel ledit mélange isomère est soumis à une amination D).

9. Procédé selon la revendication 8, **caractérisé en ce que** le produit d'amination de l'étape D) est soumis à une distillation afin de séparer le 4-amino-3,5-dichlorobenzotrifluorure.

10. Procédé selon l'une quelconque des revendications précédentes, **caractérisé en ce que** la réaction d'amination D) est réalisée avec de l'ammoniac à une température de 150°C à 350°C, éventuellement en présence d'un catalyseur d'halogénure de métal alcalin.

11. Procédé selon la revendication 10, **caractérisé en ce que** la réaction d'amination est réalisée dans un solvant organique polaire ou en utilisant le 3,4,5-TCBTF lui-même comme le solvant.

12. Procédé selon l'une quelconque des revendications précédentes, **caractérisé en ce que** le 4-chlorobenzotrichlorure, utilisé comme corps réagissant dans l'étape A), est obtenu par photochloration de parachlorotoluène avec du chlore gazeux.

13. Procédé selon l'une quelconque des revendications précédentes, **caractérisé en ce que** l'étape A) est réalisée afin d'obtenir dans le mélange des produits réactionnels une quantité de 3,4,5-trichlorobenzotrichlorure entre 20 % et 30 % en masse et une concentration de 3,4-dichlorobenzotrichlorure entre 55 et 75 % en masse par rapport à la masse totale des dérivés chlorés.

14. Procédé selon la revendication 6, **caractérisé en ce que** ladite étape de fluoration B) est réalisée avec l'utilisation d'halogénures d'antimoine comme le catalyseur.
